Europäisches Patentamt
European Patent Office
Office européen des brevets

Publication number: 0 295 341 A1

# EUROPEAN PATENT APPLICATION

Application number: 87304366.5

Int. Cl.⁴ C07D 501/56 , C07D 501/20

Date of filing: 18.05.87

Claims for the following Contracting States: AT + ES + GR.

Date of publication of application: 21.12.88 Bulletin 88/51

Designated Contracting States: AT BE CH DE ES FR GB GR IT LI LU NL SE

Applicant: Pfizer Corporation
Calle 15 1/2 Avenida Santa Isabel
Colon(PA)

Inventor: Nagakura, Isao
36-13, Takanedai Fukuzumi
Agui-cho Aichi-ken 470-22(JP)
Inventor: Nakanishi, Susumu
15, Sapia Drive
Niantic Connecticut(US)

Representative: Wood, David John et al
Pfizer Limited Ramsgate Road
Sandwich Kent CT13 9NJ(GB)

## Cephalosporins.

A cephalosporin antibiotic of the formula:-

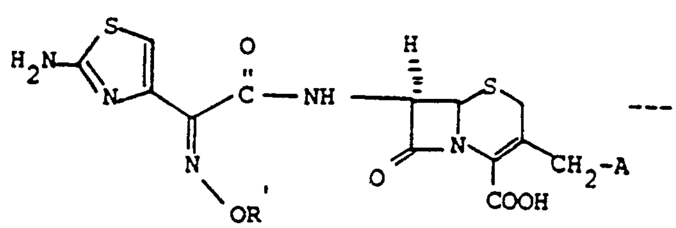

--- (II)

or a pharmaceutically-acceptable salt or in vivo hydrolysable ester thereof. wherein R' is $C_1$-$C_6$ alkyl substituted by carboxy, cyano, halo, hydroxy or a protected carboxy group; and A is phenyl or thienyl both optionally substituted by $C_1$-$C_6$ alkyl, hydroxy or halo.

EP 0 295 341 A1

# CEPHALOSPORIN COMPOUNDS

This invention relates to a new series of cephalosporin compounds which are of value as antibacterial agents. More particularly, it is concerned with novel 3-cephem compounds containing a 7-(2-(2-aminothiazol-4-yl)-2-(substituted hydroxyiminoacetamido) group at the 7-position.

The cephalosporins are a well-known family of antibiotics that have gained wide-spread use in recent years in the treatment of pathogenic infections in humans and animals. A large number of cephalosporins have been prepared by varying substituents in the 3- and 7- positions of the cephalosporin nucleus. However, the search still continues for compounds having high activity and a high degree of stability.

Although some newly-discovered cephalosporins have proven to be invaluable by virtue of their broad antibacterial spectrum against Gram-positive and Gram-negative bacteria, they do not appear to be effective against certain bacteria species e.g., Klebsiella pneumoniae, Proteus mirabillisci, Proteus vulgaris, Proteus morganii, and particularly Pseudomonas aeruginosa. These are noted as pathogens which cause serious infections in hospitals and clinics.

Japanese Patent Application 55-175,263 (Japan Kokai 57-99,592), published June 21, 1982 discloses cephalosporins of the formula:

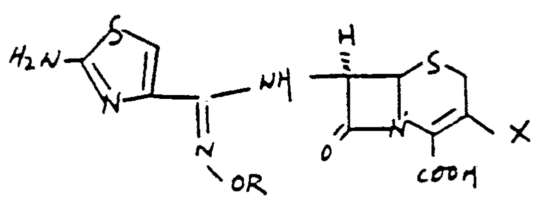

(I)

wherein R is hydrogen or alkyl and X is-$CH_2$-B wherein substituent B can be a substituted aromatic group. Only compounds having R as unsubstituted alkyl are disclosed but those having R as alkyl substituted by carboxyl, nitrile, halo, etc are not taught in said application.

U.S. Patent No. 4,409,214 discloses compounds of formula (I) wherein R may be lower alkyl being substituted by carboxy, cyano or a protected carboxyl group and X is vinyl.

In copending Japanese Patent Application 60-191,459, filed August 30, 1985 and assigned to the same assignee, compounds of formula (I) wherein R is methyl and X is

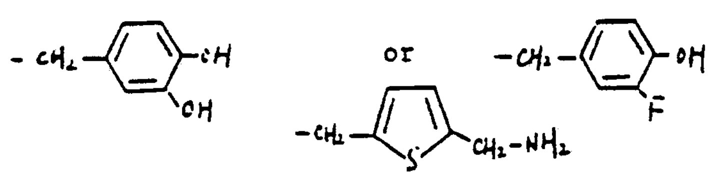

or

are disclosed.

The novel 3-cephem compounds of the present invention are represented by the following structural formula:

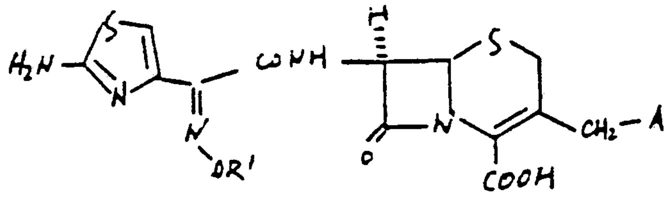

(II)

wherein R' is lower alkyl substituted by carboxyl, cyano, halogen or a protected carboxyl group; and A is phenyl, substituted phenyl, thienyl or substituted thienyl
where the substituent is selected from the group consisting of lower alkyl, hydroxyl and halo
or a pharmaceutically-acceptable salt or in vivo hydrolysable ester thereof.

The term "lower alkyl" is intended to encompass an alkyl having up to 6 carbon atoms, preferably up to 4 carbon atoms, and, where appropriate, such a group may be straight or branched chain.

The term "halo" means fluorine, chlorine, bromine or iodine.

The term "protected carboxyl group" means groups commonly employed to protect carboxyl group such as benzyl, benzhydryl, p-nitrobenzyl, etc.

Pharmaceutically-acceptable salts refer to either acid-addition salts or cationic salts. The former salts include, but are not limited to, include those with hydrochloric acid, sulfuric acid, nitric acid, phosphoric acid, citric acid, p-toluenesulfonic acid, 2-naphalenesulfonic acid, methanesulfonic acid, and the like. The latter salts include, but are not limited to, those of sodium, potassium, calcium, N, N'-dibenzylethylenediamine, triethylamine, procane, and the like.

The term "in vivo hydrolysable esters" refers to esters which are hydrolysable under physiological conditions such as acetoxymethyl, pivaloyloxymethyl, 1-ethoxycarbonyloxymethyl, 3-phthalidyl, gamma-butyrolacton-4-yl and 5-methyl-2-oxo-1,3-dioxo-4-ylmethyl. Such esters are generally used to enhance oral absorption and are now well-documented in the penicillin or cephalosporin art.

The group R' may be a group of $C_1$-$C_4$ alkyl being substituted by carboxyl, cyano or fluorine. The preferred values are of the formula:

$$-CH_2-COOH,\ -CH_2-CH_2-F,\ -CH_2-CN,\ \text{and}\ -\overset{CH_3\ \ CH_3}{C}-COOH$$

When A is substituted phenyl, it is preferably phenyl substituted by one or more substituents each independently selected from lower alkyl, hydroxyl, and halo. Examples of A as substituted phenyl are:
2-hydroxyphenyl, 3-hydroxyphenyl, 4-hydroxyphenyl, 3,5-dimethyl-4-hydroxyphenyl, 3,4-dihydroxyphenyl, 2,4-dihydroxyphenyl, 2,5-dihydroxyphenyl, 3,5-dihydroxyphenyl, and 3-fluoro-4-hydroxyphenyl.
Preferred values are 4-hydroxyphenyl, 3,5-dimethyl-4-hydroxyphenyl, 3,4-dihydroxyphenyl and 3-fluoro-4-hydroxyphenyl. The most preferred values are 3-fluoro-4-hydroxyphenyl and 3,4-dihydroxyphenyl.

When A is substituted thienyl, it is preferably thienyl substituted by one or more substituents each independently selected from lower alkyl, halo and hydroxyl. Examples of A as substituted thienyl are: 3-methyl-thien -2-yl, 4-methyl-thien-2-yl, 5-methyl-thien-2-yl. The most preferred value is 3-methyl-thien-2-yl.

Some examples of the compounds of formula (II) are: 7-(2-(2-aminothiazol-4-yl)-2-syn-carboxymethyoxyiminoacetamido)-3-(3-fluoro-4-hydroxyphenyl)methyl-$\Delta^3$-cephem-4-carboxylic acid;
7-(2-(2-aminothiazol-4-yl)-2-syn-carboxymethoxyiminoacetamido)-3-(3,4-dihydroxyphenyl)methyl-$\Delta^3$-cephem-4-carboxylic acid; and
7-(2-(2-aminothiazol-4-yl)-2-syn-(1-carboxy-1-methylethoxyimino)acetamido)-3-(3,4-dihydroxyphenyl)methyl-$\Delta^3$-cephem-4-carboxylic acid.

It is to noted that due to the geometrical isomerism of the iminoether moiety in compound (II) geometrical isomers may exist. Although only the syn isomer form is depicted in the structural formula (II) and this is preferred, the anti isomer is also possible. Both isomers and a mixture thereof are included within the scope of the present invention.

Also encompassed by the present invention is a pharmaceutical composition comprising a compound of formula (II) and a pharmaceutically acceptable diluent or carrier; and a method of treating a bacterial infection in a mammal comprising administering an antibacterially effective amount of a compound of formula (II).

The novel cephalosporin compounds of the present invention may be prepared by acylating 7-amino-3-substituted cephalosporins with an appropriately protected acylating agent and then removing the protecting group when present.

Typical route to the compounds of formula (II) is outlined below.

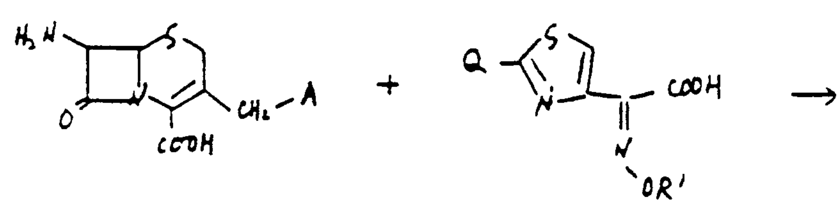

(II)

In the above formulae, R′ and A are as hereinbefore defined and Q is amino or a protected amino group. The starting material can be prepared e.g., by the methods described in Japanese Patent Appln. 59-160,998 by reacting 7-ACA with an appropriate displacing agent (i.e., AH) in the presence of Lewis acid.

The carboxylic acid group of the acylating agent is activated by any of the standard methods such as conversion to the mixed anhydride, acid chloride, acid imidazolide or activated ester. A preferred activated derivative is an acid halide e.g., the acid chloride. When the acylating agent contains a free carboxyl group, it may be blocked or protected, preferably by the use of a group which can be ultimately removed to generate the free acid group. Suitable protecting groups include t-butyl, bnezhydryl, benzyl, p-methoxybenzyl and p-nitrobenzyl. These can all be removed by conventional means. When Q is a protected amino group, the removal is conveniently carried out by the method known in the art. Suitable protecting groups for this purpose include azido, trichloroethoxycarbonyl, t-butoxycarbonyl, benzylmethoxycarbonyl, trimethylsilyl, p-methoxybenzyloxy, chloroacetyl and formamido.

Acylation reaction is typically carried out using an acid chloride of the acid at low temperature (-20°c to 0°c) in an organic solvent, e.g. dichloromethane. Other solvents which can be employed in this reaction include chloroform, diethyl ether, tetrahedrofuran, ethyl acetate, aetone, N,N-dimethylformamide, etc. For each one mole of the stating material, it is necessary to use at least one equivalent of the acylating agent, preferably 1.2 to 3 equivalents. Reaction temperature is not critical, but it is preferably in the range of -40°c to 50°c.

Any protecting groups, if present, may be removed after acylation. For example, when Q is formamido, the protecting group can be removed simply by exposing to a small amount of a strong aqeuous acid such as hydrochloric acid. When R′ contains benzhydryl, this can be removed by treating with trifluoroacetic acid or anisole/$AlCl_3$. Finally, the desired cephalosporins, can be purified by conventional methods for cephalosporin compounds, e.g. recrystallization or chromatography.

The pharmaceutically-acceptable cationic salts of the compounds of the present invention are readily prepared by standard methods. For example, one equivalent of the corresponding cationic hydroxide, carbonate or bicarbonate is combined with the carboxylic acid in an organic or aqueous solvent. Of particular value is the sodium salt. In like manner, the pharmaceutically-acceptable acid addition salts are also prepared by standard methods. If these salts precipitate, they are recovered by filtration. Alternatively, they can be recovered by evaporation of the solvent, or, in the case of aqeuous solutions, by lypohilization.

The compounds of the present invention may exist as the zwitterion which is also within the scope of the present invention.

The *in vivo* hydrolysable esters of the compounds of the present invention can also be prepared conventionally. In most instances, these esters are readily hydrolysable, upon exposure to mammalin blood or tissue, to afford the corresponding cephalosporins; they are believed to improve the absorption characteristics of cephalosporins. Thus these esters are also considered as objects of the present invention.

The utility of the compounds of formula (II) and their pharmaceutically acceptable salts and *in vivo* hydrolysable esters will be evident from their antibacterial activity. They are active against both Gram-positive and Gram-negative organisms, particularly against Gram-negative organisms such as *Escherichia coli*, *Klebsiella pneumoniae*, *Proteus vulgaris*, *Enterobacter cloacae*, *Serratia marcescers* and *Pseudomonas aeruginosa*. Minimum inhibitory concentrations (MIC's), as measured according to the well-known disc-plate method, in *in vitro* testing range from 0.1 to 100 ug/ml.

The compounds of the present invention can be administered orally or parenterally, i.e. intramuscularly, subcutaneously, intraperitoneally or intravenously, alone, or combined with a pharmaceutically-acceptable carrier. For oral administration, the compounds can be used in the form of tablets, capsules, lozenges, troches, powders, syrups, elixirs, aqueous solutions and suspensions, and the like. For parental administration, sterile solutions are usually used.

The daily dosages to be used will not differ significantly from other clinically-used cephalosporins. The prescribing physician will ultimately determine the appropriate dose for the given human subject, and this can be expected to vary according to the age, weight, and response of the individual patient as well as the nature and the severity of the patient's symptoms. The compounds of the present invention will normally be used orally at dosages in the range from 20 to about 200mg per kilogram of body weight per day, and parenterally at dosages from about 10 to about 100mg per kilogram of body weight per day, usually in divided doses. In some instances it may be necessary to use doses outside these ranges.

The following examples illustrate the invention but are not to be construed as limiting the scope thereof.

Nuclear magnetic resonance spectra (NMR) were measured at 60 $MH_z$ for solutions in perdeuterodimethyl sulfoxide (DMSO-$D_6$), and peak positions are expressed in parts per million (ppm) downfield from TMS. The following abbreviations for peak shapes are used: s, singlet, bs, broad singlet; d, doublet; t, triplet; q, quartet; m, multiplet.

7-(2-(2-Aminothiazol-4-yl)-2-syn-(2-tert-butoxy-2-oxo-ethoxyimino)acetamido)-3-(3,4-dihydroxyphenyl)-methyl-3-cephem-4-carboxylic acid

To a mixture of N,N-dimethylacetamide (DMAc) (0.7ml) and dichloromethane (5ml) was added phosphorous oxychloride (0.66ml) at 0°c. After stirring the mixture at 0°c for 30 minutes, 2-(2-aminothiazol-4-yl)-2-syn-(2-tert-butoxy-2-oxoethoxyimino)acetic acid (1.09g) was added at -15 - 20°c and stirring was continued for another 20 minutes. 7-Amino-3-(3,4-dihydroxyphenyl) methyl-3-cephem-4-carboxylic acid (0.968g) in dichloromethane (10ml) was mixed with bis(trimethylsilyl)acetamide (BSA) (3.0ml) at room temperature for one hour. This was combined with the above-mentioned solution at -30°c over 10 minutes. Stirring was continued at -30° for another one hour and then the temperature was allowed to rise to room temperature. Methanol (20ml) was added and the solution was concentrated at 30°c under reduced pressure. The residue was dissolved in ethyl acetate (140ml) and water (70ml), and insolubles were removed by decantation. The pH was adjusted to 2.5 with sodium bicarbonate. The organic layer was separated, washed with water and filtered to remove insolubles. The filtrate was dried over sodium sulfate and concentrated to solid, which was washed with ether and dried to give title compound (992mg, 54.2%): NMR 1.40( s, 9H), 3.0-3.9(m), 4.55 (bs, 2H), 5.15 (d, J=5Hz, 1H), 5.73 (dd, J=5Hz, 8Hz, 1H), 6.4-6.9 (m, 3H) 6.79 (s, 1H), 7.20 (bs), 9.54 (d, J=8Hz); infrared spectrum (KBr): 1765cm$^{-1}$.

EXAMPLE 2

7-(2-(2-Aminothiazol-4-yl)-2-syn-carboxymethoxyiminoacetamido)-3-(3,4-dihydroxyphenyl)-methyl-3-cephem-4-carboxylic acid.

Title product of the preceding Example (250mg) was dissolved in trifluoroacetic acid (1.5ml). After stirring at room temperature for one hour, the resulting solution was concentrated. The residue was tritulated in ether (5ml), filtered and dried in vacuo to give title compound as trifluoroacetic acid salt (242mg, 83.7%): NMR 3.1-4.1 (m), 4.64 (s, 2H), 5.16 (d, J=5Hz, 1H), 5.76 (dd, J=5Hz, 8Hz, 1H), 6.4-6.8 (m, 3H), 6.90 (s, 1H), 9.55 (d, J=8Hz, 1H); infrared spectrum (KBr): 1765, 1670 cm$^{-1}$.

EXAMPLE 3

7-(2-(2-Aminothiazol-4-yl)-2-syn-(2-diphenyl-methoxy-2-oxo-1,1-dimethylethoxyimino)acetamido)-3-(3,4-dihydroxyphenyl)methyl-3-cephem-4-carboxylic acid.

By the procedure of Example 1, 2-(2-aminothiazol-4-yl)-2-syn-(2-diphenylmethoxy-2-oxo-1,1-dimethylethoxyimino)acetic acid was reacted with 7-amino-3-(3,4-dihydroxyphenyl)methyl-3-cephem-4-carboxylic acid. Product was isolated as an oil in 52% yield. This product has: NMR 1.50 (s, 6H), 3.0-4.1(m), 5.15 (d, J=5Hz, 1H), 5.80 (dd, J=5Hz, 8Hz, 1H), 6.3-6.9 (m), 7.0-7.6 (m); infrared spectrum (KBr) 1765, 1740 cm$^{-1}$.

EXAMPLE 4

7-(2-(2-Aminothiazol-4-yl)-2-syn-(1-carboxy-1-methylethoxyimino)acetamido)-3-(3,4-dihydroxyphenyl)methyl-3-cephem-4-carboxylic acid

Title compound of the preceding Example (0.3g) was treated with a mixture of anisole (0.9ml) and trifluoroacetic acid 0.9ml at room temperature for two hours. The mixture was concentrated and the resulting oil was tritulated in diisopropyl ether (10ml), filtered and washed with diisopropyl ether. The solids

were put into water (20ml) and dissolved by adjusting the pH to 8.0 with aqueous sodium bicarbonate. The pH was then readjusted to 2.2 with 6N HCl. The resulting solids were filtered off, washed with water and dried in vacuo to give title compound (84.5mg, 36,3%): NMR 1.43(s, 6H), 3.0-4.2(m), 5.16 (d, J=5Hz, 1H), 5.80(dd, J=5Hz, 8Hz, 1H), 6.4-7.5(m), 8.75 (bs), 9.33 (d, J=8Hz, 1H); infrared spectrum (KBr): 1760cm⁻¹.

EXAMPLE 5

7-(2-(2-Aminothiazol-4-yl)-2-syn-cyanomethoxyimino acetamido)-3-(3-fluoro-4-hydroxyphenyl)methyl-3-cephem-4-carboxylic acid

By the procedure of Example 1, 2-(2-formamido-thiazol-4-yl)-2-syn-cyanomethoxyiminoacetic acid was reacted with 7-amino-3-(3-fluoro-4-hydroxyphenyl)methyl-3-cephem-4-carboxylic acid. 7-(2-(2-formamido-thiazol-4-yl)-2-syn-cyanomethoxyiminoacetamido)-3-(3-fluoro-4-hydroxyphenyl)methy-3- cephem-4-carboxylic acid was thus obtained in 20% yield: NMR 2.9-4.1 (m), 5.05 (s, 2H), 5.20 (d, J=5Hz, 1H), 5.80 (dd, J=5Hz, 8Hz, 1H), 6.8-7.3 (m, 3H), 7.53(s, 1H), 8.55 (s, 1H), 9.66 (bs, 1H), 9.85 (d, J=8Hz, 1H), 12.66 (s, 1H); infrared spectrum (KBr): 1770, 1675 $cm^{-1}$. This product (0.2g) in methanol (1.2ml) was treated with conc. HCl (0.12ml) at room temperature for 2.5 hours. Methanol was removed under reduced pressure and saturated sodium bicarbonate aqueous solution (10ml) was added to the resulting oil. The mixture was washed with ethyl acetate (10ml) and the aqueous layer was adjusted pH 2.5 with 6N HCl. The solids were collected by filtration, washed with water and dried in vacuo over phosphorous pentaoxide to give title product (103mg, 54.2%): NMR 3.0-4.1 (m), 5.01 (s, 2H), 5.18 (d, J=5Hz, 1H), 5.75 (dd, J=5Hz, 8Hz, 1H), 6.92 (bs), 9.80 (d, J=8Hz, 1H); infrared spectrum (KBr) $1760cm^{-1}$.

EXAMPLE 6

7-(2-(2-Aminothiazol-4-yl)-2-syn-(2-fluoroethoxyimino)acetamido)-3-(3-fluoro-4-hydroxyphenyl)methyl-3-cephem-4-carboxylic acid

By the procedure of Example 1, 2-(2-formamido-thiazol-4-yl)-2-syn-(2-fluoroethoxyimino)acetic acid was reacted with 7-amino-3-(3-fluoro-4-hydroxyphenyl)methyl-3-cephem-4-carboxylic acid. 7-(2-(2-formamidothiazol-4-yl)-2-syn-2-(fluoroethoxyimino)-acetamido)-3-(3-fluoro-4-hydroxyphenyl) methyl-3-cephem-4-carboxylic acid was thus obtained in 45% yield: NMR 3.0-5.2 (m), 5.16 (d.J=5Hz, 1H), 5.73 (dd, J=5Hz, 8Hz, 1H), 6.78 (s, 1H), 6.8-7.3 (m), 9.55 (d, J=8Hz, 1H); infrared spectrum (KBr): 1770, $1670cm^{-1}$.

EXAMPLE 7

7-(2-(2-Aminothiazol-4-yl)-2-syn-(carboxymethoxyimino)acetamido)-3-(4-hydroxyphenyl)methyl-3-cephem-4-carboxylic acid

By the procedure of Example 1, 2(2-aminothiazol-4-yl)-2-syn-(2-tert-butoxy-2-oxoethoxy)imino)acetic acid was reacted with 7-amino-3-(4-hydrophenyl)methyl-3-cephem-4-carboxylic acid. The crude product in ethyl acetate solution was treated with diphenyldiazomethane. The mixture was concentrated to give solids. After washing with petroleum ether, column chromatography on silica gel using 2:3 ethyl acetate: dichloromethane afforded diphenylmethyl 7-(2(2-aminothiazol-4-yl)-2-syn-(2-tert-butoxy-2-oxoethoxyimino)-acetamido-3-(4-hydroxyphenyl)methyl-3-cephem-4-carboxylate in 36% yield: NMR 1.39(s, 9H), 4.53 (s, 2H), 5.23 (d, J=5Hz, 1H), 5.83 (dd, J=5Hz, 8Hz, 1H), 6.5-7.7 (m); infrared spectrum (KBr): 1770, 1740 $cm^{-1}$.

Following the procedure of Example 2. this product (0.40g) was converted by deprotection of carboxylic groups to title compound (265mg, 73%): NMR 3.1-4.2 (m). 4.63 (s, 2H). 5.15 (d. J=5Hz, 1H), 5.73 (dd. J=5Hz, 8Hz. 1H), 6.67 (d, J=8Hz. 1H). 6.90 (s. 1H), 7.09 (d. J=8Hz, 1H); infrared spectrum (KBr): 1765, 1670 $cm^{-1}$.

EXAMPLE 8

7-(2-(2-Aminothiazol-4-yl)-2-<u>syn</u>-(2-t-butoxy-2-oxo-ethoxyimino)acetamido)-3-benzyl-3-cephem-4-carboxylic acid

By the procedure of Example 1. 2-(2-aminothiazol-4-yl)-2-syn-(2-tert-butoxy-2-oxoethoxyimino)acetic acid was reacted with 7-amino-3-phenylmethyl-3-cephem-4-carboxylic acid to yield title compound: NMR 1.40 (s, 9H), 3.0-4.2 (m. 4H), 4.53 (s, 2H), 5.18 (d, J=5Hz, 1H), 5.75 (dd, J=5Hz, 8Hz, 1H), 6.78 (s, 1H). 7.27 (s, 5H), 9.42 (d, J=8Hz, 1H); infrared spectrum (KBr): 1765 $cm^{-1}$.

EXAMPLE 9

7-(2-(2-Aminothiazol-4-yl)-2-syn-carboxymethoxyiminoacetamido)-3-benzyl-3-cephem-4-carboxylic acid

Following the procedure of Example 2. title product of the preceding Example was converted to 7-(2-(2-aminothiazol-4-yl)-2-syn-carboxymethoxy iminoacetamido)-3-benzyl-3-cephem-4-carboxylic acid: NMR 3.1-4.2 (m), 4.60 (s, 2H), 5.20 (d, J=5Hz, 1H). 5.76 (dd, J=5Hz, 8Hz, 1H), 6.85 (s, 1H), 7.30 (s, 5H). 9.55 (d, J=8Hz, 1H); infrared spectrum (KBr): 1770, 1670 $cm^{-1}$.

EXAMPLE 10

7-(2-Aminothiazol-4-yl)-2-syn-(1-carboxy-1-methylethoxyimino)acetamido)-3-(3-fluoro-4-hydroxyphenyl)-methyl-3-cephem-4-carboxylic acid

By the procedure of Example 1. 2-(2-aminothiazol-4-yl)-2-syn-(2-diphenylmethoxy-2-oxo-1,1-dimethylethoxyimino)acetic acid was reacted with 7-amino-3-(3-fluoro-4-hydroxyphenyl)methyl-3-cephem-4-carboxylic acid to give.

7-(2-(2-aminothiazol-4-yl)-2-syn-(2-diphenyl-methoxy-2-oxo-1,1-dimethylethoxyimino)acetamido)-3-(3-fluoro-4-hydroxyphenyl)methyl-3-cephem-4-carboxylic acid. Following the procedure of Example 4, this product was converted to title compound: NMR 1.45 (s, 6H), 3.1-4.0 (m), 5.18 (d,J=5Hz. 1H), 5.80 (dd. J=5Hz, 8Hz, 1H), 6.88 (s), 6.8-7.2 (m), 9.48 (d, J=8Hz, 1H); infared spectrum (KBr): 1760, 1670, 1630 $cm^{-1}$.

EXAMPLE 12

7-(2-(2-Aminothiazol-4-yl)-2-syn-carboxymethoxyiminoacetamido)-3-(3,5-dimethyl-4-hydroxyphenyl)methyl-3-cephem-4-carboxylic acid

By the procedure of Example 1, 2-(2-aminothiazol-4-yl)-2-syn-(2-tert-butoxy-2-oxoethoxyimino)acetic acid was reacted with 7-amino-3-(3,5-dimethyl-4-hydroxyphenyl)methyl-3-cephem-4-carboxylic acid to give 7-(2-(2-aminothiazol-4-yl)-2-syn-(2-tert-butoxy-2-oxoethoxyimino)acetamido-3-(3,5-dimethyl-4-hydroxyphenyl)methyl-3-cephem-4-carboxylic acid. Following the procedure of Example 2, this product was converted by deprotection to title compound: NMR 2,12 (s, 6H), 3.0-4.0 (m), 4.63 (s, 2H), 5.15 (d, J=5Hz, 1H), 5.95 (dd, J=5Hz, 8Hz, 1H), 6.8-7.1 (bs, 3H), 9.53 (d, J=8Hz, 1H); infrared spectrum (KBr): 1770, 1670, 1200 $cm^{-1}$.

EXAMPLE 13

7-(2-(2-Aminothiazol-4-yl)-2-syn-carboxymethoxyiminoacetamido)-3-(3-methylthien-2-yl)methyl-3-cephem-4-carboxylic acid

By the procedure of Example 1, 2-(2-aminothiazol-4-yl)-2-syn-(2-tert-butoxy-2-oxoethoxyimino) acetic acid was reacted with 7-amino-3-(3-methylthien-2-yl)methyl-3-cephem-4-carboxylic acid to give 7-(2-(2-aminothiazol-4-yl)-2- syn-(2-tert-butoxy-2-oxoethoxyimino)acetamido-3-(3-methylthien-2-yl)methyl-3-cephem-4-carboxylic acid. Following the procedure of Example 2, this product was converted by deprotection to title compound: NMR 2.16 (s, 3H), 3.0-4.1 (m), 5.15 (d, J=5Hz, 1H), 5.87 (dd, J=5Hz, 8Hz, 1H), 6.84 (s, 1H), 6.83, 7.28 (AB J=4Hz), 9.53 (d, J=8Hz, 1H); infrared spectrum (KBr): 1770 1670 $cm^{-1}$.

EXAMPLE 14

7-(2-(2-Aminothiazol-4-yl)-2-syn-(2-tert-butoxy-2-oxoethoxyimino)acetamido)-3-(3-methylthien-2-yl)methyl-3-cephem-4-carboxylic acid

By the procedure of Example 1, 2-(2-aminothiazol-4-yl)-2-syn-(1,2-diphenylmethoxy-2-oxo-1,1-dimethylethoxyimino)acetic acid was reacted with 7-amino-3-(3-methylthien-2-yl)methyl-3-cephem-4-carboxylic acid to give 7-(2-(2-aminothiazol-4-yl)-2-syn-(1,2-diphenylmethoxy-2-oxo-1,1-dimethylethoxyimino)-acetamido-3-(3-methylthien-2-yl)methyl-3-cephem-4-carboxylic acid. Following the procedure of Example 4, this product was converted by deprotection to title compound: 1.42 (s, 6H), 2.15 (s, 3H), 3.2-4.1 (m), 5.16 (d, J=5Hz, 1H), 5.78 (dd, J=5Hz, 8Hz, 1H), 6.76 (s, 1H), 6.82, 7.25 (AB, J=5Hz), 9.38 (d, J=8Hz, 1H); infrared spectrum (KBr): 1760 $cm^{-1}$.

EXAMPLE 15

7-(2-(2-Aminothiazol-4-yl)-2-syn-(2-fluoroethoxyimino)acetamido)-3-(3-methylthien-2-yl)methyl-3-cephem-4-carboxylic acid.

By the procedure of Example 1, 2-(2-formamido-thiazol-4-yl)-2-syn-(2-fluoroethoxyimino acetic acid was reacted with 7-amino-3-(3-methylthien-2-yl)-3-cephem-4-carboxylic acid. 7-(2-(2-formamido-thiazol-4-yl)-2-syn-(2-fluoroethoxyimino)acetamido)-3-(3-methylthien-2-yl)methyl-3-cephem-4- r-carboxylic acid was thus obtained: NMR 2,18 (s, 3H), 3.1-5.2 (m), 5.16 (d, J=5Hz), 5.76 (dd, J=5Hz, 8Hz, 1H), 6.83, 7.25 (AB, J=5Hz), 7.42 (s, 1H), 8.51 (s, 1H); infrared spectrum (KBr): 1770, 1675 $cm^{-1}$. Following the same deprotection procedure as described in Example 5, this product was converted to title compound: NMR 2.16 (s, 3H), 3.0-5.2 (m), 5.16 (d, J=5Hz, 1H), 5.75 (dd, J=5Hz, 8Hz, 1H), 6.77 (s, 1H), 6.82, 7.23 (AB, J=5Hz), 9.60 (d, J=8Hz, 1H); infrared spectrum (KBr): 1760 $cm^{-1}$.

EXAMPLE 16

7-(2-(2-Aminothiazol-4-yl)-2-syn-cyanomethoxyiminoacetamido)-3-(3-methylthien-2-yl)methyl- $^{3}$-cephem-4-carboxylic acid

By the procedure of Example 1, 2-(2-formamido-thiazol-4-yl)-2-syn-cyanomethoxyiminoacetic acid was reacted with 7-amino-3-(3-methylthien-2-yl)methyl-c-cephem-4-carboxylic acid. 7-(2-(2-formamidothiazol-4-yl-2-syn-cyanomethoxyiminoacetamido)-3-(3-methylthien-2-yl)methyl-3-cephem-4-carboxylic acid was thus obtained: NMR 2.19 (s. 3H), 3.1-4.3 (m). 5.08 (s. 2H). 5.20 (d, J=5Hz, 1H), 5.81 (dd, J=5Hz, 8Hz, 1H), 6.85, 7.28 (AB, J=5Hz), 7.55 (s, 1H). 8.55 (s, 1H), 9.88 (d, J=8Hz, 1H), 12.8 (s, 1H); infrared spectrum (KBr): 1770, 1675 cm$^{-1}$.

Following the same deprotection procedure as described in Example 5, this product was converted to title compound: NMR 2.16 (s, 3H). 3.0-4.1 (m), 4.98 (s, 2H), 5.15 (d, J=5Hz, 1H), 5.72 (dd, J=5Hz, 8Hz, 1H), 6.86 (s, 1H), 6.82, 7.26 (AB, J=5Hz), 9.76 (d, J=8Hz, 1H); infrared spectrum (KBr): 1760 cm$^{-1}$.

PREPARATION A

7-Amino-3-(4-hydroxyphenyl)methyl-3-cephem-4-carboxylic acid

To a mixture of 2.6-di-tert-butylphenol (57.2g) and 17% $BF_3$-acetonitrile solution (280ml) was added 7-aminocephalosporanic acid (50g) at -20 °c. The resulting solution was warmed to 0 °c and poured into ice-water (500ml). The pH was adjusted to 3.5 with sodium carbonate. The precipitated solid was isolated, then repulped in water-diethyl ether (1:1), and dried in vacuo to afford 33.4g (44%) of 7-amino-3-(4-hydroxy-3,5-di-tert-butylphenyl)methyl-3-cephem-4-carboxylic acid: NMR 1,37 (s, 18H), 3.0-4.5 (m, 4H), 4.72, 4.99 (d, J=4.6Hz), 7.06 (s, 2H); infrared spectrum (KBr): 1770 cm$^{-1}$.

To a mixture of this product (19.2g), anisole (25ml) and toluene (45ml) was added aluminium chloride (20g) at 0 °c. The resulting mixture was warmed to room temperature, stirred for 5 hours, and poured into ice-water (200ml). The pH was adjusted to 3.5 with sodium bicarbonate. The precipitated solid was isolated by filtration, washed with water and then toluene, and dried in vacuo to afford title compound (11.75g, 85%): NMR 2,9-4.0 (m. 4H), 4.75 (d, J=5Hz, 1H), 5.05 (d, J=5Hz, 1H), 6.70 (d, J=8Hz, 2H), 7.10 (d, J=8Hz, 2H); infrared spectrum (KBr): 1780 cm$^{-1}$.

PREPARATION B

7-Amino-3-phenylmethyl-3-cephem-4-carboxylic acid

The procedure of preparation A, substituting benzene for 2,6-di-tert-butylphenol gave title compound: NMR 3.10, 3.53 (AB, J=18Hz), 3.55, 3.85 (AB, J=16Hz), 4.72 (d, J=5Hz, 1H), 5.00 (d, J=5Hz, 1H), 7.30 (bs, 5H).

By the same method the other 7-amino-substituted phenylmethyl or thienylmethyl-3-cephem-4-carboxylic acid derivatives used through Examples are prepared.

## Claims

1. A compound of the formula:-

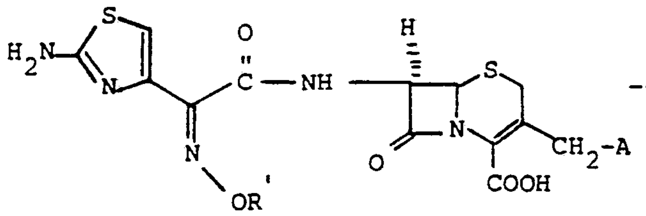

--- (II)

or a pharmaceutically-acceptable salt or in vivo hydrolysable ster thereof, wherein R′ is $C_1$-$C_6$ alkyl substituted by carboxy, cyano, halo, hydroxy or a protected carboxy group; and A is phenyl or thienyl both optionally substituted by $C_1$-$C_6$ alkyl, hydroxy or halo.

2. A compound according to claim 1 wherein R′ is selected from:

$-CH_2-COOH$, $-CH_2-CH_2-F$, $-CH_2-CN$ and 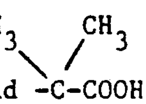

3. A compound according to claim 1 or 2 wherein A is selected from: 4-hydroxyphenyl, 3,5-dimethyl-4-hydroxyphenyl, 3,4-dihydroxyphenyl and 3-fluoro-4-hydroxyphenyl.

4. A compound according to claim 3, said compound being 7-(2-(2-aminothiazol-4-yl)-2-syn-carboxymethoxyiminoacetamido)-3-(3-fluoro-4-hydroxyphenyl)methyl-$\Delta^3$-cephem-4-carboxylic acid.

5. A compound according to claim 3, said compound being 7-(2-(2-aminothiazol-4-yl)-2-syn-carboxymethoxyiminoacetamido)-3-(2,4-dihydrophenyl)methyl-$\Delta^3$-cephem-4-carboxylic acid.

6. A compound according to claim 3, said compound being 7-(2-(2-aminothiazol-4-yl)-2-syn-(1-carboxy-1-methylethoxyimino)acetamido)-3-(3,4-dihydroxyphenyl)methyl-$\Delta^3$-cephem-4-carboxylic acid.

7. A pharmaceutical composition comprising a compound as claimed in any one of the preceding claims, or a pharmaceutically-acceptable salt or in vivo hydrolysable ester thereof, and a pharmaceutically acceptable diluent or carrier.

8. A compound, salt or ester thereof as claimed in any one of claims 1 to 6 for use as a medicament.

9. The use of a compound, salt or ester thereof as claimed in any one of claims 1 to 6 for the manufacture of an antibacterial agent.

Claims for the following contracting States: AT, ES and GR

1. A process for preparing a cephalosporin antibacterial compound of the formula:-

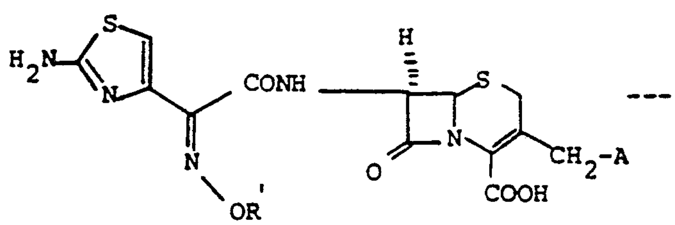

--- (II)

wherein R′ is $C_1$-$C_6$ alkyl substituted by carboxy, cyano, halogen or a protected carboxy group;
and A is phenyl or thienyl both optionally substituted by $C_1$-$C_6$ alkyl, hydroxy or halo;
or a pharmaceutically-acceptable salt thereof or an in vivo hydrolyzable ester thereof;
characterised in that a 7-amino-3-substituted cephalosporin compound of the formula:-

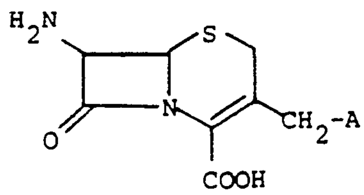

where A is as defined above,
is acylated with an acid of the formula:-

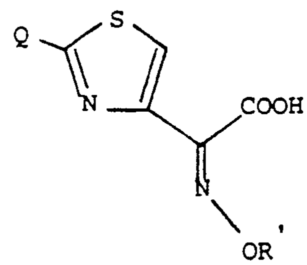

or with its functional equivalent as an acylating agent, wherein Q is amino or a protected amino group and R' is as defined above;

followed by, when Q is a protected amino group, removal of the protecting group;

said process also being followed by, optionally, conversion of the product into a pharmaceutically acceptable salt or in vivo hydrolyzable ester.

2. A process according to claim 1, characterised in that a mixed anhydride, acid chloride, acid imidazolide or activated ester of the acid is used as the acylating agent.

3. A process according to claim 2, characterised in that the acylation is carried out with an acid chloride.

4. A process as claimed in any one of the preceding claims, characterised in that the reaction is carried out at a temperature of from -20 °C to 0 °C in an organic solvent.

5. A process according to claim 4, characterised in that the organic solvent is dichloromethane, chloroform, diethyl ether, tetrahydrofuran, ethyl acetate, acetone, or N,N-dimethylformamide.

6. A process according to any oen of claims 1 to 5, characterised in that R' is $-CH_2-CH_2-COOH$, $-CH_2-CH_2-F$, $-CH_2-CH_2-CN$ or $-C(CH_3)_2-COOH$; and that A is 4-hydroxyphenyl, 3,5-dimethyl-4-hydroxyphenyl, 3,4-dihydroxyphenyl or 3-fluoro-4-hydroxyphenyl.

7. A process for preparing a pharmaceutical composition, characterised by mixing a compound of the formula (II) as defined in claim 1, or a pharmaceutically acceptable salt or in vivo hydrolysable ester thereof, with a pharmaceutically acceptable diluent or carrier.

European Patent Office

# EUROPEAN SEARCH REPORT

Application Number

EP 87 30 4366

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 061 234 (BEECHAM)<br>* Pages 30-33, claims *<br>--- | 1-9 | C 07 D 501/56<br>C 07 D 501/20 |
| D,A | DE-A-3 137 854 (TOYAMA)<br>* Pages 28-30; page 182, table 16; pages 184,193,195 * & JP-A-57 99 592 (Cat. D)<br>--- | 1-9 | |
| D,A | PATENT ABSTRACTS OF JAPAN, vol. 11, no. 246 (C-439)[2693], 11th August 1987; & JP-A-62 51 688 (TAITO PFIZER K.K.) 06-03-1987<br>----- | 1-9 | |
| | | | TECHNICAL FIELDS SEARCHED (Int. Cl.4) |
| | | | C 07 D 501/00 |

The present search report has been drawn up for all claims

| Place of search | Date of completion of the search | Examiner |
|---|---|---|
| THE HAGUE | 08-01-1988 | LUYTEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document